# EUROPEAN PATENT APPLICATION

(11) **EP 1 659 169 A1**
(43) Date of publication of application: **24.05.2006**
(21) Application number: 04745419.4
(22) Date of filing: 28.05.2004
(51) Int. Cl.: C12M 1/00, C12M 3/00

(54) **CULTURE TREATING APPARATUS AND AUTOCULTURING APPARATUS**

(30) Priority: 02.07.2003 JP 2003270524; 30.04.2004 JP 2004135638
(71) Applicant: Olympus Corporation, Tokyo 151-0072 (JP)
(72) Inventor: HIBINO, Hiroki, Hachioji-shi, Tokyo 1930811 (JP); HOSHIKAWA, Noriyuki, Hachioji-shi, Tokyo1920917 (JP)
(74) Representative: von Hellfeld, Axel
(86) International application number: PCT/JP2004/007383
(87) International publication number: WO 2005/003283

(57) **Abstract**

Contamination into other culture containers by airborne matter which had been spattered when a predetermined treatment is performed on a specimen in a culture container is prevented. There is provided a culture treatment apparatus comprising: a treatment chamber in which is stored a treatment device (19) which opens a lid (3b) of a culture container (3) storing a specimen to perform a predetermined treatment on the specimen inside of the culture container (3); and a sheet member (45) arranged below the culture container (3), which cover the top surface of the treatment device (19).

## Description

### [Technical Field]

The present invention relates to a culture treatment apparatus and an automatic culture apparatus.

### [Background Art]

As a conventional automatic culture apparatus, there is known an automatic culture apparatus comprising: a fixed-type storage shelf which is capable of storing a plurality of culture containers; and a carrier device which is horizontally, vertically, and rotatably movable (for example, refer to Patent Document 1).
This automatic culture apparatus is configured such that, the storage shelf placed in a culture chamber comprises a plurality of vertically aligned small chambers, and one culture container is respectively stored in each small chamber to perform culturing. Then during the culturing or after completing the culturing, the carrier device is operated so as to take out the culture containers one by one from the small chamber, or to store culture containers into the small chamber.
In this automatic culture apparatus, each small chamber is not sealed but is opened to inside the culture chamber, and the culture chamber is maintained in a constant culture condition.

Moreover, in this automatic culture apparatus, the culture container that has been taken out from the small chamber, is sent out from the openable/closable opening provided in the wall surface of the culture chamber, to the outside of the culture chamber, so as to perform treatments such as exchanging media, by various treatment devices placed outside of the culture chamber.

### [Patent Document 1]

Japanese Unexamined Patent Application, First Publication No. 2002-262856 (FIG. 1 and the like).

### [Disclosure of Invention]

In the automatic culture apparatus, treatments such as injecting media into the culture container and discharging media from the culture container are performed by a medium injecting needle and a medium discharging needle, by the operation of an injecting pump and a discharging pump. However, if a medium is released forcefully from the medium injecting needle, there is concern that when the media is being injected into the culture container, splashes of medium are spattered, thus contaminating the various treatment devices. Moreover, there is also concern that the media which has been once drawn by the medium discharging needle may drip from the tip of the medium discharging needle. In particular, if the spattered medium contains a part of the cells, there is concern that the splashes are dried with time and become airborne, causing of contamination into other culture containers.

In order to avoid such concerns, it is necessary to clean the space where the treatment has been performed, each time after completing the treatment step for each culture container. However, automation of the cleaning step is difficult, and since the cleaning step requires a cleaning operation by manpower, there is concern of decreasing the efficiency.

The present invention takes the above problems into consideration with an object of providing a culture treatment apparatus and an automatic culture apparatus capable of preventing contamination into other culture containers by airborne matter which had been spattered when a predetermined treatment is performed on a specimen in a culture container.

In order to achieve the above object, the present invention provides the following solutions.
The present invention provides a culture treatment apparatus comprising: a treatment chamber in which is stored a treatment device which opens a lid of a culture container storing a specimen to perform a predetermined treatment on the specimen inside of the culture container; and a sheet member arranged below the culture container, which covers a top surface of the treatment device.
According to this invention, by means of the treatment device in the treatment chamber, a predetermined treatment, such as the supply/discharge of medium, is performed on the specimen in the culture container. In this case, since the lid of the culture container is opened, at the time of treatment, there is a likelihood of spattering of specimen in the culture container to the outside of the culture container. However, even in this case, since the sheet member which covers the top surface of the member is arranged below the culture container, the spattered specimen is captured on the sheet member. Consequently by merely exchanging the sheet member, the inside of the culture treatment apparatus can be readily cleaned.

Moreover, the present invention provides a culture treatment apparatus comprising: a supply roll made from said sheet member in belt shape rolled into a roll shape; a sheet supply device which sends out the sheet member from the supply roll; and a sheet collection device which collects the used sheet member.

According to this invention, the sheet supply device operates to supply the sheet member from the supply roll that has been rolled in a roll shape, so as to cover the top surface of the treatment device, and the sheet collection device operates to collect the used sheet member. As a result, since the specimen that has been spattered from inside the culture container and then captured by the sheet member is collected together with the sheet member, it can be prevented from becoming airborne in the culture treatment apparatus due to drying out, or the like.

Furthermore, the present invention provides a culture treatment apparatus wherein the sheet collection device comprises a roll-up device which rolls up the sheet member into a roll shape.
According to this invention, since the roll-up device operates to roll up the used sheet member into a roll shape, it becomes possible to efficiently collect the sheet member.

Moreover, the present invention provides a culture treatment apparatus wherein the sheet member is made from a liquid absorbable material.
According to this invention, since a specimen dropping from the inside of the culture container is absorbed into the sheet member, the specimen can be collected while being held by the sheet member without spilling outside, when being collected by the sheet collection device.

Furthermore, the present invention provides a culture treatment apparatus comprising an antiseptic solution supply device which impregnates an antiseptic solution into the sheet member.
According to this invention, since the antiseptic solution supply device operates to impregnate the antiseptic solution into the sheet member, a specimen that had dropped from the inside of the culture container and has then been captured by the sheet member, is sterilized, preventing bacteria from reproducing.

Moreover, the present invention provides a culture treatment apparatus wherein the sheet member is made of a material having an antibacterial action.
According to this invention, due to the effect of the sheet member itself, bacteria and the like in the captured specimen are prevented from reproducing.

Furthermore, the invention may comprise a tray supply device which supplies a tray member onto a stage where a culture container is mounted, when a predetermined treatment is applied to a specimen in the culture container by opening a lid of the culture container storing the specimen.
Since the tray supply device operates to supply a tray member onto the stage, then in a state where the culture container is mounted on the tray member, the lid of the culture container storing the specimen is opened, and a predetermined treatment is applied to the specimen in the culture container. At the time of the treatment, there is a likelihood of spattering of the specimen in the culture container to the outside of the culture container. However, even in this case, the spattered specimen or the like is captured by the tray member. Consequently, by merely exchanging the tray member, cleanliness of the inside of the culture treatment apparatus can be kept high.

Moreover, the invention may comprise a tray collection device which collects a used tray member after a treatment on a specimen in a culture container is completed, and the culture container is removed from the tray member.
Since the tray collection device operates to collect the used tray member from which the specimen has been collected, a culture container containing the next specimen is mounted on a new tray member, preventing the previous specimen from being mixed thereinto.

Furthermore, in the above-described invention, the tray member may be provided with a fixing portion which fixes the culture container on the tray member.
Since the culture container is kept from being moved on the tray member by the fixing portion, it becomes possible to stir the specimen in the culture container by vibrating or shaking together with the tray member. Moreover, when a treatment is performed by an automatic machine such as a robot, it becomes possible to arrange the culture container in an always fixed position, so that there is no need for a complicated device such as a device for detecting the position of the culture container. As a fixing portion, a concave portion for engaging the culture container, or convex portions such as pins provided on the tray member so as to be in contact with the periphery of the culture container may be employed.

In the above-described invention, the tray member is preferably provided with a peripheral wall which surrounds the culture container when it is mounted thereon.
By providing the tray member with the peripheral wall, the specimen leaking from the culture container is dammed up by the peripheral wall on the tray member. Consequently, further leakage from the tray member and spattering can be prevented.

Moreover the present invention provides an automatic culture apparatus comprising: a culture chamber which stores a culture container storing a specimen, in a manner where it can be put in/taken out; a treatment chamber which is arranged outside of the culture chamber, and which stores a treatment device which opens a lid of the culture container to perform a predetermined treatment on the specimen inside of the culture container; and a carrier mechanism which carries the culture container between the treatment chamber and the culture chamber, wherein at least either one of the treatment chamber and the culture chamber comprises sheet member arranged below the culture container, which covers a top surface of the treatment device.

According to this invention, the specimen that has been cultured in the culture chamber is carried to the treatment chamber by the operation of the carrier mechanism, and then subjected to a predetermined treatment by the treatment device in the treatment chamber. Moreover, the specimen that has been subjected to the predetermined treatment in the treatment chamber is put back into the culture chamber by the operation of the carrier mechanism, so as to continue the culturing step. As a result, the specimen is automatically cultured. In this case, since in at least either one of the treatment chamber and the culture chamber, the top surface of the treatment device is covered by the sheet member arranged below the culture container, even if the specimen drops from the inside of the culture container for some reason, the specimen is captured by the sheet member. Consequently, inside of the automatic culture apparatus can be readily cleaned.

According to the culture treatment apparatus and the automatic culture apparatus of this invention, since a specimen containing liquid that has been spattered from the inside of the culture container is captured by the sheet member, the liquid can be prevented from being adhered onto the top surface of the treatment device. As a result, by merely exchanging the sheet member, the inside of the culture treatment apparatus and the automatic culture apparatus can be maintained in a clean condition. Moreover, the cleaning step inside of the culture treatment apparatus and the automatic culture apparatus can be simplified, demonstrating an effect of further increasing the percent of automization of the culture treatment including the maintenance work.

### [Brief Description of Drawings]

[FIG. 1] A perspective view showing an automatic culture apparatus according to an embodiment of the present invention.
[FIG. 2] A vertical cross-sectional view schematically showing a first space of the automatic culture apparatus of FIG. 1.
[FIG. 3] A vertical plan view schematically showing the first space of the automatic culture apparatus of FIG. 1.
[FIG. 4] A perspective view showing an example of a culture container used in the automatic culture apparatus of FIG. 1.
[FIG. 5] A perspective view showing a centrifugal separator placed in a culture treatment apparatus of the automatic culture apparatus of FIG. 1.
[FIG. 6] A perspective view showing a tip supply device placed in the culture treatment apparatus of the automatic culture apparatus of FIG. 1.
[FIG. 7] A perspective view showing a reagents supply device placed in the culture treatment apparatus of the automatic culture apparatus of FIG. 1.
[FIG. 8] A perspective view showing a horizontal transfer mechanism placed in the culture treatment apparatus of the automatic culture apparatus of FIG. 1.
[FIG. 9] A schematic view showing an antiseptic solution supply device for a sheet member.
[FIG. 10] A perspective view showing a modified example of the automatic culture apparatus of FIG. 1.
[FIG. 11] A perspective view for describing the supply and the collection of tray members with respect to a stage in the automatic culture apparatus of FIG. 10.
[FIG. 12] A perspective view showing a case where a cover is placed over the tray supply section of FIG. 11.
[FIG. 13] A perspective view showing a case where pins are arranged instead of concavities in the tray member of FIG. 10.

### [Best Mode for Carrying Out the Invention]

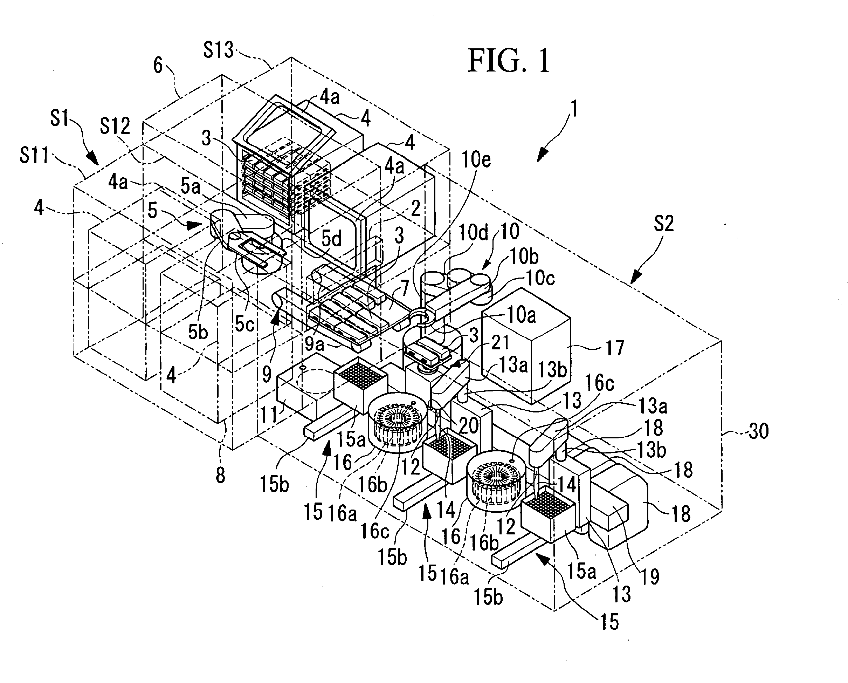

A culture treatment apparatus and an automatic culture apparatus according to embodiments of the present invention, are described with reference to FIG. 1 to FIG. 8.
The automatic culture apparatus 1 according to the present embodiment is sealed up by transparent wall materials to enable observation from the outside, and comprises a first space S1 and a second space S2 (treatment chamber) which are communicated with each other through a shutter 2.

A total of four culture chambers 4 containing culture containers 3 are arranged two each in opposite spaces S11 and S13 of the first space S1. A center space S12 comprises a carrier robot (carrier mechanism) 5 for moving the culture containers 3. On the top of the center space S12 is provided an air cleaner 6 which sends out a clean downwards airflow for purifying the air inside the center space S12.
The four culture chambers 4 are set apart with each of two chambers arranged side by side, facing the other two chambers with the doors 4a in between, so that the doors 4a are arranged facing the center space S12.

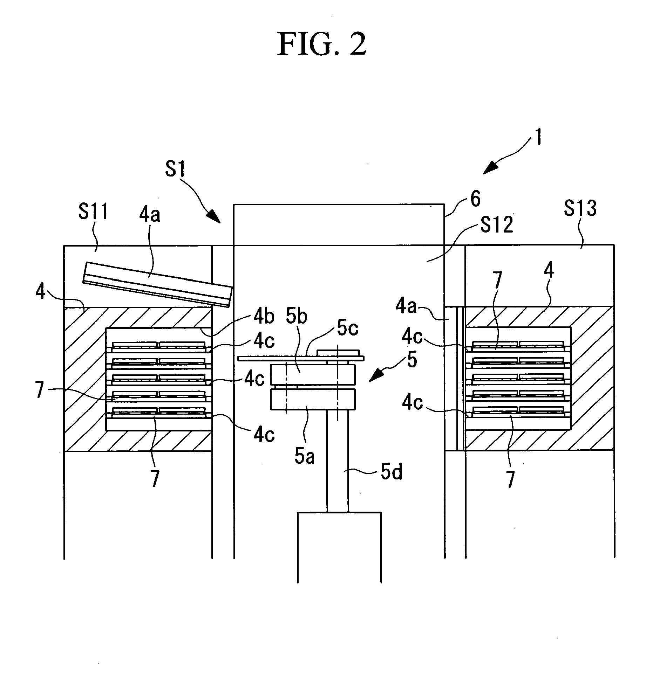

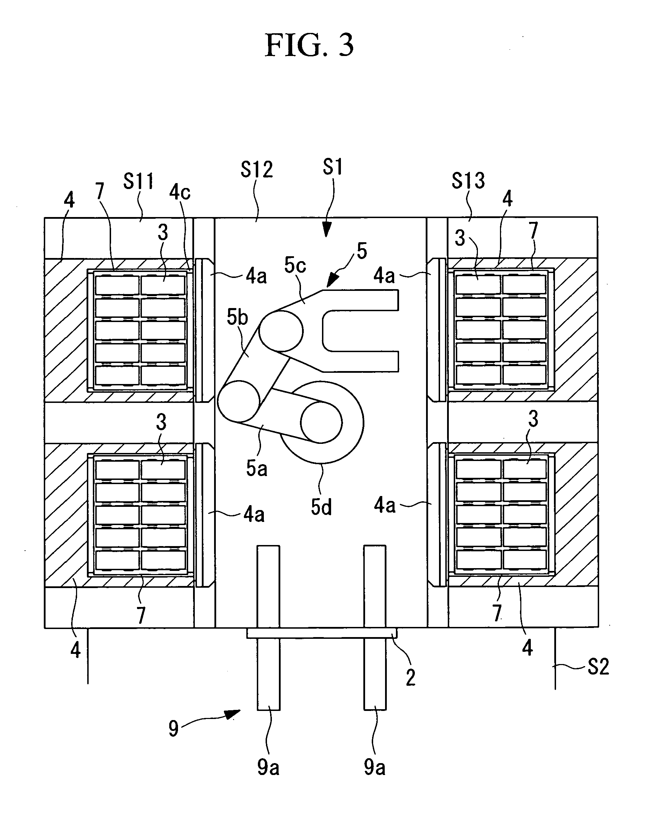

As shown in FIG. 2 and FIG. 3, the respective culture chambers 4 have openings 4b on one side, comprising doors 4a which can open/close the openings 4b. On the left and right side walls facing the opening 4b are provided a plurality of rail-shaped tray holding members 4c at corresponding height positions so that a plurality of tiers of trays 7 can be vertically stored while being spanned across to the left and right pairs of respective tray holding members 4c. Inside of the respective culture chambers 4 is kept at a predetermined culture condition, for example, at a temperature of 37±0.5°C, a humidity of 100%, and a CO₂ concentration of 5%. The tray holding members 4c are not limited to a rail-shape, and may be in any shape as long as they can support the trays 7 in the manner where the trays can be put in/taken out.

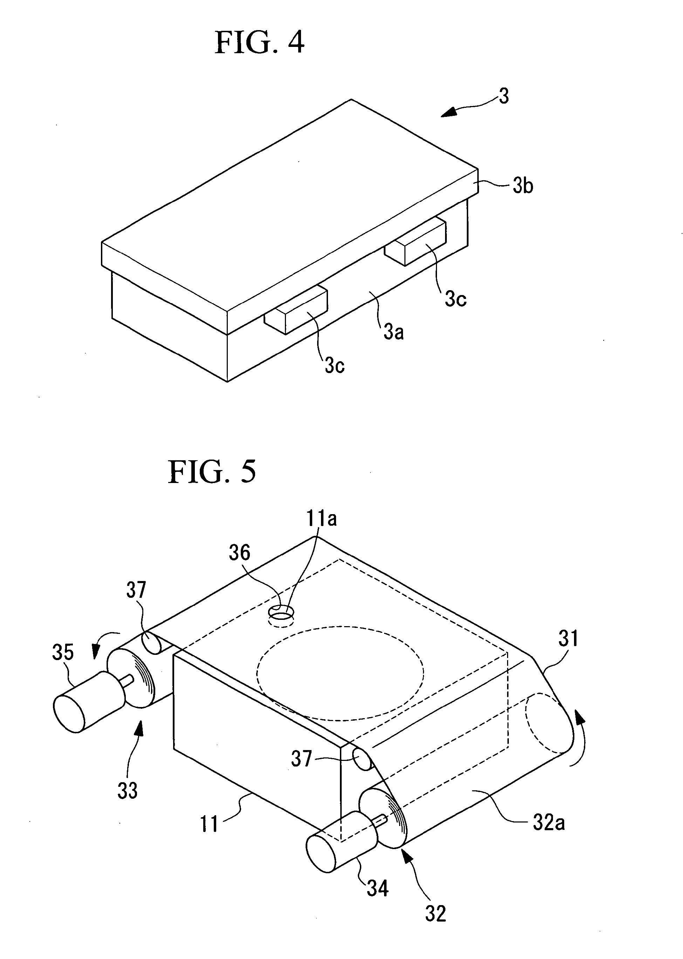

Each tray 7 is designed so that a plurality of, for example ten culture containers 3 can be mounted while being aligned. As shown in FIG. 4, each culture container 3 comprises a container main body 3a and a lid body 3b provided on the top of the container main body 3a. On the left and right sides of the container main body 3a are provided projections 3c which can be hooked by a hand in a second space described later.

At the bottom of each culture chamber 4 is arranged a stocker 8 for containing a plurality of unused culture containers 3 in a condition mounted on the tray 7. The stocker 8 has a door which can be opened/closed, on a side surface on the opposite side to the door of the culture chamber 4, facing the outside of the first space S1. The door is formed in a size allowing opening of the whole one side of the stocker 8.

The carrier robot 5 is arranged approximately in the center of the space between the four culture chambers 4. The carrier robot 5 comprises: a first arm 5a which is horizontally rotatable; a second arm 5b which is connected to an extremity of the first arm 5a in a rotatable manner around the vertical axis; a hand 5c which is attached to an extremity of the second arm 5b in a rotatable manner around the vertical axis, and which does not have any mechanism to spoil the environment in the culture chamber, such as an actuator or a transmission mechanism; and an elevating/lowering mechanism 5d which can elevate/lower the first arm 5a, the second arm 5b, and the hand 5c. Due to this configuration, the carrier robot 5 has a horizontal operating range where it can access all trays 7 in the four culture chambers 4, and can deliver the trays 7 onto a conveyer 9 that is arranged between the first space S1 and the second space S2 and spans across the shutter 2.

The conveyer 9 comprises two endless belts 9a which are arranged left and right with a spacing greater than the width dimension of the hand 5c of the carrier robot 5, and so that the trays 7 can be spanned across the endless belts 9a and mounted thereon. Moreover, the carrier robot 5 has a vertical operating range such that it can access all trays 7 in the culture chambers 4, and can access at least the trays 7 on the top tier in the stockers 8.
The belt 9a is not limited to an endless belt.

The hand 5c is formed in a flat shape extending in the horizontal direction so that the tray 7 can be mounted thereon, and is formed with a thickness dimension such that it can be inserted into a gap between the trays 7 stored in the culture chambers 4. The hand 5c is designed so that by elevating from the condition of being inserted into a gap between the trays 7, the trays 7 are lifted up from below by two arms and taken out from the tray holding member 4c, and the trays 7 can be stably held.

The second space S2 is configured with a culture treatment apparatus 30. The culture treatment apparatus 30 comprises: a handling robot 10 which handles a culture container 3 on a tray 7 carried from the first space S1 by the conveyer 9 while the shutter 2 is opened; a centrifugal separator (treatment device) 11 which separates cells from media in the culture containers 3; two dispenser robots 13 which are horizontally rotatable and vertically movable, and comprises an electric pipette 12 for dispensing various liquids such as serum and reagents; three tip supply devices (treatment device) 15 which store a plurality of disposable tips 14 to be attached to the tip end of the electric pipettes 12 of these dispenser robots 13, and can supply them to within the operating range of dispenser robots 13; a tip collection section (not shown) which disposes and collects the used tips 14; a reagents supply device (treatment device) 16 which stores various liquids such as serum and reagent in a plurality of containers; a microscope (treatment device) 17 by which the behavior of cells in the culture container 3 can be observed; a plurality of storage tanks 18 which respectively store waste liquids disposed due to exchange of respective reagents and media; a horizontal transfer mechanism (treatment device) 19 which moves the culture containers 3 so that the culture containers 3 can be transferred between the conveyer 9 and the respective robots 10 and 13; and a shaker 21 attached to a slider 20 of the horizontal transfer mechanism 19, on which the received culture containers 3 are mounted and shaken.
The second space S2 is also provided with an air cleaner (not shown) which sends out a clean downward air flow for purifying the air inside the second space S2.

The handling robot 10 is a horizontal multi-articulated robot which horizontally and vertically moves the holding hand 10a for handling a culture container 3. For example, in the example shown in FIG. 1 it comprises three horizontal arms 10b, 10c, and 10d that are connected to each other, and an elevating/lowering mechanism 10e which elevates/lowers these horizontal arms 10b to 10d. Moreover, on the extremity of the horizontal arms 10b to 10d, in addition to the holding hand 10a which holds a culture container 3, there is provided an electric pipette (not shown) to and from which a tip 14 for putting in and taking out cells and media with respect to the culture container 3 can be attached and detached, and a lid body open/close hand (not shown) which hooks the lid body 3b of the culture container 3 to open/close it.

The handling robot 10 is designed; to open/close the lid body 3b of the culture container 3 on the tray 7 that has been carried by the conveyer 9, to hold and carry the culture container 3 so as to supply it to the shaker 21 and the microscope 17, to exchange the tip 14 on the tip end of the electric pipette, and to put the cell containing media taken out from the culture container 3 into the centrifugal separator 11. Consequently, for the handling robot 10, various devices such as the conveyer 9, the shaker 21, the microscope 17, the tip supply device 15, the tip collection device, and the centrifugal separator 11, are arranged within its operating range.

The centrifugal separator 11 is designed to rotate the cell-containing media that have been supplied from the handling robot 10, at low speed, so that the high density cells that have been floating in the media can be separated from the media and settled. As shown in FIG. 5, the centrifugal separator 11 is provided with a sheet member 31 which covers the top surface, a sheet supply device 32 which supplies the sheet member 31, and a sheet collection device 33 which collects the sheet member 31.

The sheet member 31 is formed in a long length band shape having a width dimension greater than the width dimension of the centrifugal separator 11. The sheet member 31 is made of, for example, a liquid absorbable cloth. Moreover, the opposite ends of the sheet member 31 are rolled into a roll shape. The sheet supply device 32 comprises a sending motor 34 which drives in a direction to send out the sheet member 31 from a roll shaped supply roll 32a arranged on one end. The sheet collection device 33 comprises a roll-up motor 35 which rolls up the sheet member 31, on the other end of the sheet member 31. The sheet member 31 is formed with a hole 36 in a position which corresponds to an input port 11a for input of cells into the centrifugal separator 11 when the sheet member 31 is sent out for a predetermined length and stopped, so that the tip 14 attached to the tip end of the electric pipette of the handling robot 10 can be inserted into the input port 11a of the centrifugal separator 11 through the hole 36.
Reference symbol 37 in the drawing denotes an idle roller which holds the sheet member 31 in a stretched state at a distance above the centrifugal separator 11.

The dispenser robot 13 comprises an arm 13a which is horizontally rotatable and comprises an electric pipette 12 for detachable attachment of the tips 14 onto the tip end thereof, and an elevating/lowering mechanism 13b which elevates/lowers the arm 13a. The dispenser robot 13 is designed to supply media and various reagents into the culture container 3 that has been carried by the horizontal transfer mechanism 19. Consequently, for the dispenser robot 13, various devices such as the shaker 21 on the horizontal transfer mechanism 19, the tip supply device 15, the tip collection section, and the reagents supply device 16, are arranged within its operating range.

The tip supply device 15 is configured such that, a plurality of tips 14 are stored in an array in a container 15a which is opened upward, with the attachment holes for the electric pipette 12 facing upward, so that the tips 14 can be attached to the tip end of the electric pipette 12 by merely inserting the electric pipette 12 from above, at any time when the handling robot 10 or the dispenser robot 13 require new tips 14. The container 15a is attached to the transfer mechanism 15b so that it can be reciprocated in a direction intersecting with the direction of movement of the electric pipette 12 by the handling robot 10 or the dispenser robot 13. As a result, the electric pipette 12 can access all tips 14 in the container 15a.

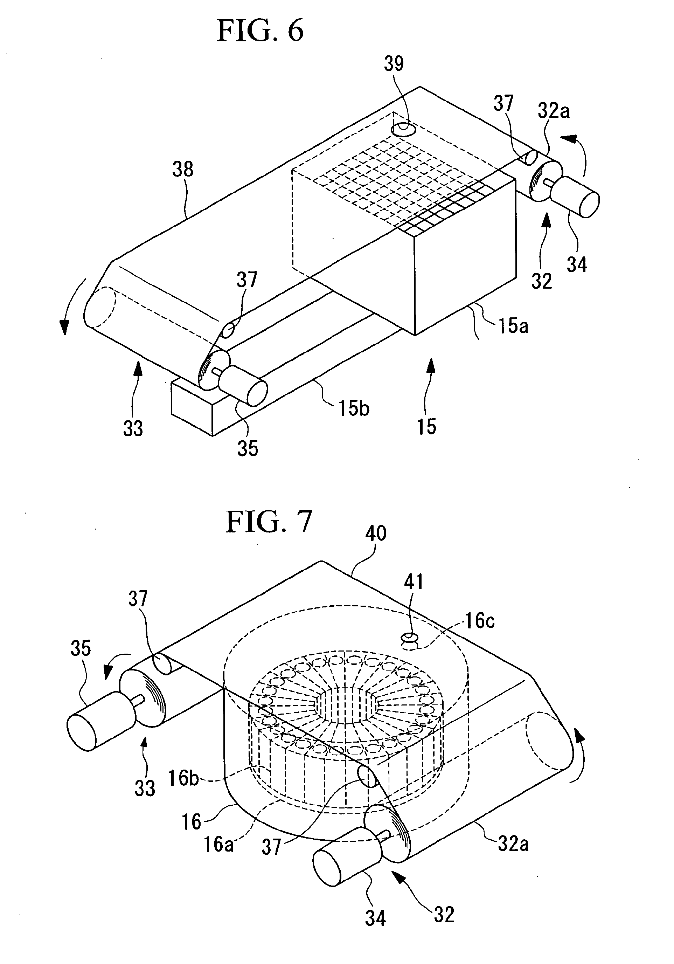

As shown in FIG. 6, the tip supply device 15 comprises a sheet member 38 which covers the top surface of the container 15a, and a sheet supply device 32 and a sheet collection device 33 which are similar to the above. The sheet member 38 is placed so as to cover the top surface of the container 15a over the whole movement range of the transfer mechanism 15b. As a result, the top opening of the container 15a is covered by a sheet member 38 when the container 15a is in any position, preventing dust from being adhered onto the tips 14 inside of the container 15a. Moreover, the sheet member 38 is provided with a hole 39 that is arranged above the position of the next tip 14 to be taken out from the container 15a. As a result, the electric pipette 12 can take out the tip 14 that is arranged underneath, through the hole 39.

The tip collection device (not shown) comprises a holding device for holding a tip 14, at the entrance of the collection container, which is designed to hold a tip 14 that has been used by the handling robot 10 or the dispenser robot 13, when the tip 14 is inserted into the holding device. In this state, the handling robot 10 or the dispenser robot 13 moves the electric pipette 12, to thereby remove the used tip 14 from the tip end of the electric pipette 12, and collect this into the collection container.

As shown in FIG. 1, the reagents supply device 16 comprises: for example, a horizontally rotatable table 16a which is stored in a cylindrical casing; and a plurality of canister-shaped reagents containers 16b, each having a fan shaped bottom, which are arranged in the circumferential direction mounted on the table 16a. Various reagents are stored in the respective reagents containers 16b. Examples of such stored reagents include MEM (Minimal Essential Medium) or DMEM (Dulbecco's Modified Eagle Medium) constituting a medium required for culturing cells, serums such as FBS (Fetal Bovine Serum) or human serum, proteases such as trypsin that exfoliates cells in the culture container 3, growth factors such as cytokine that grow cells at the time of culturing, differentiated induction factors such as dexamethasone that differentiate cells, antibiotics such as penicillin, hormone drugs such as estrogen, and nutritions such as vitamins.

On the top surface of the casing of the reagents supply device 16 is provided an insertion port 16c into which the dispenser robot 13 inserts the tip 14 on the tip end of the electric pipette 12. The insertion port 16c is placed within the operating range of the dispenser robot 13. Moreover, the top surface of each reagents container 16b comprises an opening (not shown) arranged in a position which corresponds with the insertion port 16c. As a result, by rotating the table 16a to arrange the opening of the reagents container 16b vertically below the insertion port 16c of the casing, the dispenser robot 13 can insert the tip 14 on the tip end of the electric pipette 12 from above into the reagents container 16b, so as to draw reagents that have been stored inside. The reason why two of each reagents supply devices 16 and dispenser robots 13 are provided, is to separately handle drug solutions such as trypsin which is common to all specimens, and liquids such as serum which is peculiar to each specimen.

As shown in FIG. 7, above the reagents supply device 16 is provided a sheet member 40, and a sheet supply device 32 and a sheet collection device 33 which are similar to the above. The sheet member 40 is provided with a hole 41 that is arranged above the insertion port 16c of the casing. The dispenser robot 13 can insert the tip 14 on the tip end of the electric pipette 12 through the hole 41 and into the insertion port 16c, so as to draw up reagents in the casing.

The microscope 17 is designed to be used when counting the number of cells in the culture container 3 during the culturing step, or at the time of medium exchange. The microscope 17 is configured so that the XY stage adjustment, working distance adjustment, magnification change, and the like can all be performed by remote control operation. The configuration may be such that, by arranging an ocular lens facing the outside of the second space S2, the state of cells in the culture container 3 can be visually observed from the outside of the automatic culture apparatus 1.

The storage tank 18 is designed to store for example, MEM and PBS (phosphate buffered saline) that can be commonly used for all specimens, and to supply this as necessary into the reagents container 16b in the reagents supply device 16. Moreover, some storage tanks 18 may be used as a waste liquid tank to store waste media and the like, that are discharged at the time of medium exchange.

The horizontal transfer mechanism 19 comprises a slider 20 which is horizontally movable by a rectilinear transfer mechanism. The shaker 21 is mounted on the slider 20 so that the culture container 3 mounted on the shaker 21 can be moved from the conveyer 9 to within the operating range of the dispenser robots 13.

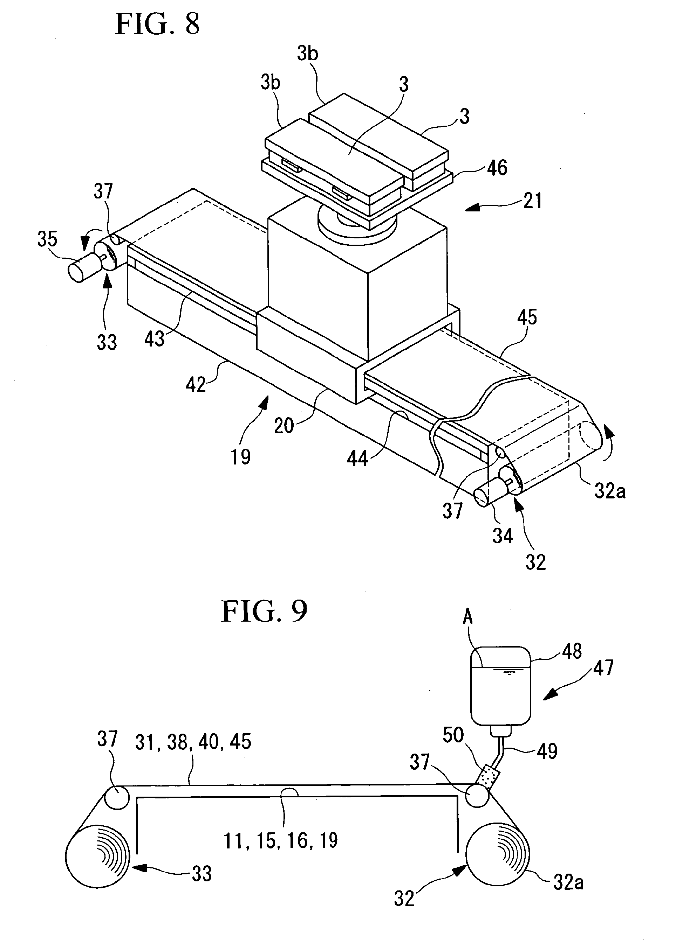

As shown in FIG. 8, the slider 20 of the horizontal transfer mechanism 19 holds around a top plate 43 covering the top surface of the horizontal transfer mechanism main body 42, and is connected to a rectilinear transfer mechanism (not shown) such as a ball screw or a linear guide inside of the horizontal transfer mechanism main body 42 through a slit 44 provided on the side faces. A belt shaped sheet member 45 is inserted through a gap between the top plate 43 and the slider 20 in the horizontal transfer mechanism 19, and is arranged so as to cover the top surface of the top plate 43 along the longitudinal direction of the horizontal transfer mechanism main body 42. The sheet member 45 is rolled into a roll shape on a sheet supply device 32 and a sheet collection device 33 that are similar to the above, and are arranged on the opposite ends.

The shaker 21 comprises a holding mechanism (not shown) on which the culture container 3 that has been transferred from the inside of the tray 7, on the conveyer 9 is mounted and held, and a vibration device (not shown) which applies vibration to the culture container 3. The vibration device is for example a device to reciprocatingly shake the culture container 3 within a predetermined angle range. As such a vibration device, a device which applies ultrasonic vibration or a device which applies horizontal vibration may be employed.
The various devices of the automatic culture apparatus 1 according to the present embodiment are connected to a controller (not shown). The controller is designed to control the sequence of respective steps, the timing of the operation, and the like, and to record keep the operation history, and the like.

Hereunder is a description of the operation of the culture treatment apparatus 30 and the automatic culture apparatus 1 according to the present embodiment configured in this manner.
In order to culture cells using the automatic culture apparatus 1 according to the present embodiment, firstly a bone marrow fluid that has been collected from a patient is put in a centrifuge container (not shown) and placed in the centrifugal separator 11. This step may be performed by an operator, or by the handling robot 10. As a result, by the operation of the centrifugal separator 11, high density myeloid cells are collected from the bone marrow fluid.

The collected myeloid cells are put into the culture container 3 by the handling robot 10. At this time, by the operation of the conveyer 9, ten empty culture containers 3 mounted on the tray 7 are fed out from the first space S1 to the second space S2. The handling robot 10 opens two lid bodies 3b among the culture containers 3 that have been fed out, and then operates the holding hand 10a to hold the two culture containers 3, and thereby transfer and mount them onto the shaker 21. A robot which opens the lid body 3b may be separately provided. As a result, the lid body 3b can be opened just before the treatment, so that the likelihood of foreign bodies entering into the container main body 3a can be reduced.

When the tip supply device 15 operates the transfer mechanism 15b to place the unused tips 14 within the operating range of the handling robot 10, the handling robot 10 takes an unused tip 14 from the tip supply device 15 and attaches it to the tip end of the electric pipette.
In this state, the handling robot 10 is operated so that the tip 14 on the tip end of the electric pipette comes into contact with the myeloid cells that have been collected in the centrifugal separator 11. Then, the electric pipette is operated to draw the myeloid cells into the tip 14. By operating the handling robot 10, the drawn myeloid cells are put into the culture containers 3 that have been transferred and mounted on the shaker 21 with the lid bodies 3b opened.
Once the myeloid cells have been put into the culture containers 3, the handling robot 10 carries the tip 14 to the tip collection section, and detaches the tip 14.

In this case, during the period while the handling robot 10 is carrying the myeloid cells from the centrifugal separator 11 to the culture container 3, or during the period while the tip 14 is being moved from the culture container 3 having the myeloid cells put thereinto, to the tip collection device, there is concern that myeloid cell containing liquid may drop from the tip end of the tip 14, or that liquid which has splashed at the time of being put from the tip 14 into the culture container 3 may be spattered out of the culture container 3. According to the culture treatment apparatus 30 and the automatic culture apparatus 1 of the present embodiment, the myeloid cell containing liquid that has been spattered out of the culture container 3 in this manner, drops onto the top surfaces of various treatment devices 11, 15, 16, and 19 placed below the culture container 3.

According to the culture treatment apparatus 30 of the present embodiment, since the top surfaces of various treatment devices, in particular the centrifugal separator 11, the tip supply device 15, the reagents supply device 16, and the horizontal transfer mechanism 19, are covered by the sheet members 31, 38, 40, and 45, then liquid dropping from the tip 14 and the culture container 3 is captured by the sheet members 31, 38, 40, and 45 and absorbed therein, thus preventing the liquid from being adhered onto the various treatment devices 11, 15, 16, and 19. Moreover, by periodically operating the sheet supply device 32 and the sheet collection device 33, the used sheet members 31, 38, 40, and 45 having the liquid adhered thereon are rolled up, and new sheet members 31, 38, 40, and 45 are supplied. Consequently, the situation where the liquid is dried with time and becomes powdery so that it becomes airborne in the culture treatment apparatus 30, is prevented.
As a result, the number of times of the cleaning operation in the culture treatment apparatus 30 can be reduced, and the percent of automization of the culture treatment including the maintenance work can be further increased.

Next, by operating the horizontal transfer mechanism 19, the culture container 3 having the myeloid cells put thereinto, is horizontally moved together with the shaker 21, and placed within the operating range of the respective dispenser robots 13. The dispenser robot 13 operates the electric pipette 12 with an unused tip 14 that has been received from the tip supply device 15 attached to its tip end, so as to draw the proper amount of DMEM, serum, or various reagents from the inside of the reagents container 16b of the reagents supply device 16, and then carry it to above the culture container 3 and pour it into the culture container 3. The serum and respective reagents are drawn after exchanging for an unused tip 14 supplied from the tip supply device 15 at each time of drawing the respective reagents. As a result, in the culture container 3, myeloid cells are present while being mixed in an appropriate medium. In order to evenly distribute the myeloid cells in the medium, the shaker 21 may be operated to apply vibration to each culture container 3.

In this case, at the time of pouring various reagents into the culture container 3 using the reagents supply device 16, or at the time of vibrating by the shaker 21, there is a likelihood of spattering of cell containing liquid from the inside of the culture container 3 to outside of the culture container 3. According to the culture treatment apparatus 30 and the automatic culture apparatus 1 of the present embodiment, since the various treatment devices 11, 15, 16, and 19 placed below the culture container 3 are covered by the sheet members 31, 38, 40, and 45, then the spattered liquid can be captured and periodically collected by the sheet members 31, 38, 40, and 45, thereby preventing proliferation of the cells, or the cells becoming airborne due to drying out.

Then, the culture container 3 for which all treatments have been completed is brought back to within the operating range of the handling robot 10, by the operation of the horizontal transfer mechanism 19. The handling robot 10 then brings the culture container 3 back onto the tray 7, with the lid body 3b covering the container main body 3a.
After completing predetermined treatments on all culture containers 3 on the tray 7, the conveyer 9 is operated to thereby insert the culture container 3 mounted on the tray 7 to inside the center space S12 of the first space S1 from the second space S2.

In this state, the carrier robot 5 is operated to lift the tray 7 by the hand 5c. Then, once the tray 7 has been carried to the front of the culture chamber 4 which stores the tray 7, the door 4a of the culture chamber 4 is opened and the tray 7 is inserted through the door and put onto the empty tray holding member 4c by the carrier robot 5. Then, by closing the door 4a again, the culture condition inside the culture chamber 4 is kept at a predetermined level so as to culture cells. Needless to say, the sequence of putting in the myeloid cells and the various reagents of DMEM, and serum, and drawing them out may be appropriately modified.

Moreover, at the time of exchanging the medium and exchanging the containers, similarly to the above, by the operation of the carrier robot 5 placed outside of the culture chambers 4, the culture container 3 in the culture chamber 4 is taken out together with the tray 7, and delivered from the first space S1 to the second space S2. In the second space S2, trypsin is poured into the culture container 3, and in a state where the cells in the culture container 3 are exfoliated, the handling robot 10 is operated to put the culture container 3 into the centrifugal separator 11 so as to collect only the necessary part such as mesenchymal cells. Other treatment steps are similar to the above.

Then, by performing culturing steps for a predetermined period through a plurality of times of medium exchange and container exchange, the mesenchymal cells are proliferated to a sufficient cell number. Whether the sufficient cell number is achieved or not is determined by operating the handling robot 10 to carry the culture container 3 with mesenchymal cells adhered the bottom, to the microscope 17 and measuring. On the tray 7, the culture containers 3 of the same specimen may be mounted, or the culture containers 3 of different specimens may be mixed. Moreover, on the shaker 21, the culture containers 3 of the same specimen may be mounted, or the culture containers 3 of different specimens may be mixed.

In this manner, the automatic culture apparatus 1 according to the present embodiment enables automatic culturing of a sufficient number of mesenchymal cells from the bone marrow fluid that has been collected from a patient. After obtaining sufficient mesenchymal cells, a material for repairing biological tissues such as calcium phosphate, or a differentiation-inducing factor such as dexamethasone can be put into the culture container 3 to continue the culturing step again, so as to manufacture a product for repairing biological tissues which can be filled into a defect in biological tissues.

In this case, according to the automatic culture apparatus 1 of the present embodiment, a mechanical section for taking out the culture container 3 is not present in the culture chambers 4. That is, the tray holding member 4c which supports the tray 7 in the mounted state is only provided in the culture chambers 4, and all mechanical sections for taking out the culture container 3 are put together in the carrier robot 5 that is placed outside of the culture chambers 4. Moreover, the carrier robot 5 is designed so as to be able to be completely withdrawn to outside of the door 4a of the culture chamber 4, after completing the operation of putting in/taking out the trays 7.

Consequently, in the state where the door 4a is closed, any mechanical section is not present in the culture chamber 4, and any dust that can be generated by the operation of such a mechanical section is not present at all. Moreover, the culture chamber 4 is kept for example at a temperature of 37±0.5°C, a humidity of 100%, and a CO₂ concentration of 5%. However, since the mechanical section is not present, there is no problem of corrosion and the like generated even in such an environment. Moreover, even in the state where the door 4a is opened, only the tip end of the hand 5c of the carrier robot 5 is inserted into the culture chamber 4, and no rotating mechanism nor sliding mechanism substantially enters into the culture chamber 4. Consequently, dust is kept from entering the culture chamber 4, improving the cleanliness of the inside of the culture chamber 4.
The culture chambers 4 may be configured by items used for culturing such as a CO₂ incubator, a multigas incubator, an incubator, and a cooling box, or a combination thereof.

Furthermore, the automatic culture apparatus 1 according to the present embodiment comprises an air cleaner 6 on the top of the center space S12 where the carrier robot 5 is set. Therefore the cleanliness of the inside of the center space S12 where the carrier robot 5 is present is always maintained. Consequently, even when the door 4a of the culture chamber 4 is opened, it becomes possible to minimize dust flowing into the culture chamber 4.
Consequently, according to the automatic culture apparatus 1 of the present embodiment, there is an effect of reducing the likelihood of contamination of the cells being cultured, by dust and the like, so that healthy cells can be cultured.

The present invention is not limited to the structure shown in the above embodiment. That is, the shape and number of the culture chambers 4, the form and number of the carrier robot 5, the handling robot 10, and the dispenser robot 13, the form and number of various devices, and so on, are not limited and may be optionally set according to the condition of application.
Moreover, as a growth factor, in addition to cytokine, for example, substances which contribute to growth such as platelet concentrate, BMP, EGF, FGF, TGF-β, IGF, PDGF, VEGF, and HGF, and the complexes thereof, may be employed. Furthermore, as an antibiotic, in addition to penicillin, any antibiotic such as cephems, macrolides, tetracyclines, fosfomycins, aminoglycosides, and new quinolones may be employed.

Moreover, as the material for repairing biological tissues, instead of calcium phosphate, any material that is compatible with biological tissues may be employed, and bioabsorbent material is more preferable. In particular, biocompatible porous ceramics, collagen, polylactic acid, polyglycolic acid, hyaluronic acid, or combinations thereof may be used. Moreover, a metal such as titanium may be used. The material for repairing biological tissues may be in granular form, or block form.

Furthermore in the culture treatment apparatus 30 and the automatic culture apparatus 1 according to the present embodiment, the top surfaces of the centrifugal separator 11, the tip supply device 15, the reagents supply device 16, and the horizontal transfer mechanism 19 are covered by the sheet members 31, 38, 40, and 45. However the configuration is not limited to this, and the top surfaces of other treatment devices, for example, the stage 46 of the shaker 21, or the X-Y stage of the microscope 17, on which the culture container 3 is mounted, may be covered by a sheet member that is similar to the above. Moreover, as the sheet supply device 32 and the sheet collection device 33, a type which rolls up in a roll shape is employed. However it may be a type which supplies folded sheet members 31, 38, 40, and 45 while unfolding them, or a type which collects them into a collection container (not shown) without rolling them up. Furthermore, the holes 36, 39, and 41 through which the tip 14 can be inserted are provided in the sheet member, so as to match the holes 36, 39, and 41 with the insertion ports 11a, 16c, and the like that have been provided in the treatment devices 11, 15, and 16. However, instead of this, the sheet members 31, 38, 40, and 45 may be configured so that they can be pierced by the electric pipette 12 and the like, so as to exchange tips and draw reagents while opening holes in optional positions by the electric pipette 12 and the like.

Furthermore, as shown in FIG. 9, there may be provided an antiseptic solution supply device 47 which impregnates an antiseptic solution A such as ethyl alcohol into the sheet members 31, 38, 40, and 45. The antiseptic solution supply device 47 comprises: a reservoir tank 48 which stores the antiseptic solution A; and a sponge member 50 which is provided on an outlet pipe 49 of the reservoir tank 48, and is made to contact with the sheet members 31, 38, 40, and 45. As a result, the antiseptic solution A in the reservoir tank 48 gradually leaks out through the sponge member 50 so as to be impregnated into the sheet members 31, 38, 40, and 45. By having such a configuration there is an advantage in that, even though cell containing liquid which is spattered from the inside of the culture container 3 is captured by the sheet members 31, 38, 40, and 45, bacteria are prevented from reproducing by sterilizing using the antiseptic solution A.

Moreover, as the horizontal transfer mechanism 19, a structure which moves the slider 20 with respect to the horizontal transfer mechanism main body 42 was used for description. However, the sheet member 45 itself that is moved above the horizontal transfer mechanism main body 42 may be used like a conveyer, by mounting the culture container 3 thereon. In this case, since the culture container 3 is not positioned with respect to the sheet member 45, dislocation may occur. Therefore, the position of the culture container 3 may be confirmed by a position detection device (not shown) such as a camera.

Moreover, the places covered by the sheet members 31, 38, 40, and 45 are not limited to those within the culture treatment apparatus 30, and various devices in the first space S1 may be covered.
Furthermore, instead of the antiseptic solution supply device 47, the sheet members 31, 38, 40, and 45 themselves may have an antibacterial action. By so doing, bacterial propagation can be prevented, similarly to with the antiseptic solution supply device 47, and the percent of automization can be further increased, since the supply of the antiseptic solution A is unnecessary.

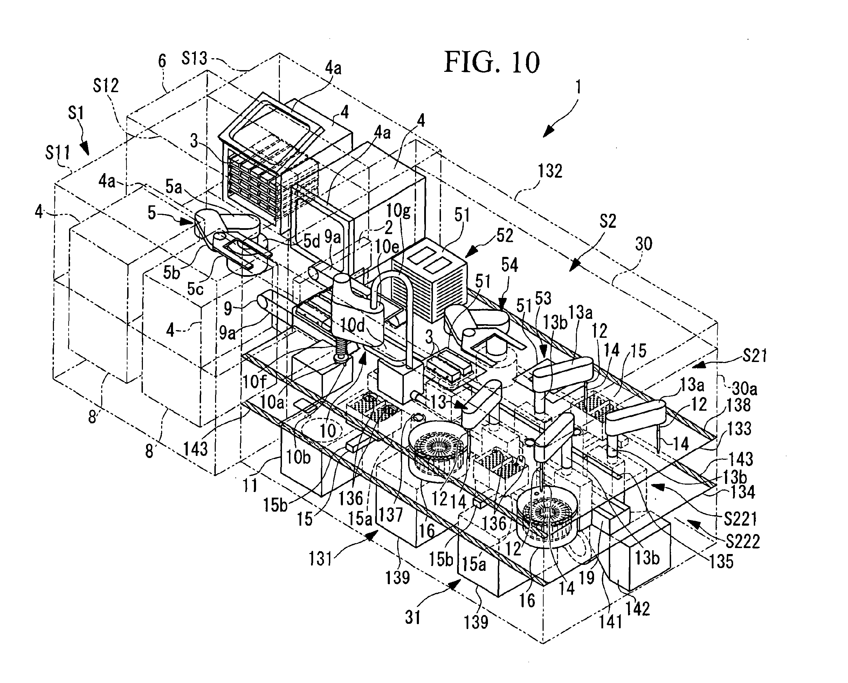

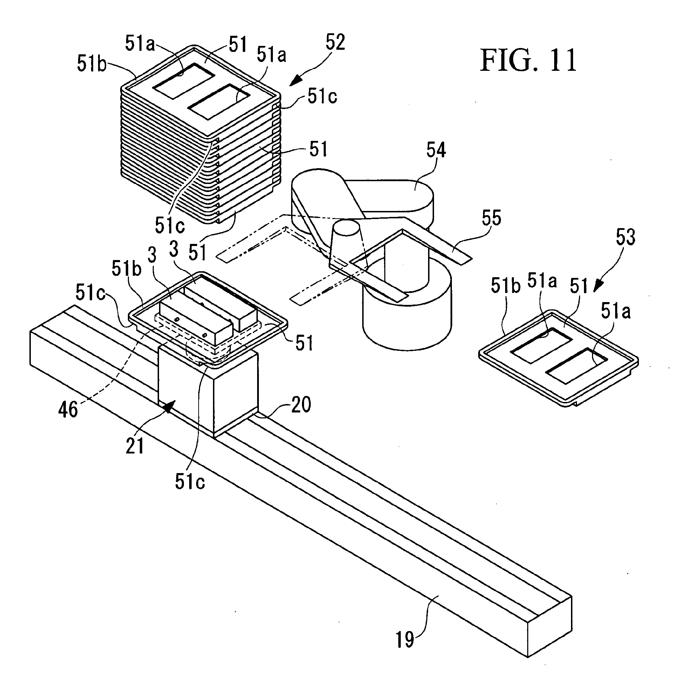

As shown in FIG. 10 and FIG. 11, on the stage 46 of the shaker 21 which is moved by the horizontal transfer mechanism 42, may be detachably fixed tray members 51 on which the culture containers 3 are mounted.
FIG. 10 shows an example where the second space S2 of the culture treatment apparatus 30 is partitioned into three spaces S21, S221, and S222 one above the other, by partitions 133 and 134. The space S21 is a space where treatments are performed on the culture containers, and high cleanliness is maintained. The space S221 is a space where mainly mechanical sections, the tip supply device 15, and the reagents supply device 16 are arranged.

In the space S222, the centrifugal separator 11, the storage tank 18, the waste container 139 of the tip collection section 131, and an exhaust fan 141 are arranged. The exit of the exhaust fan 141 is provided with a filter 142 such as a HEPA filter, so as to purify the exhaust air. The downward air flow in the space S21 from an air cleaner 132 is drawn by the exhaust fan 141 and guided into the space S222 through vent holes 138 and 143, then exhausted to the outside by the exhaust fan 141.

Reference symbol 135 denotes a long hole for connecting the stage 46 to the horizontal transfer mechanism 19 in the space S221 so as to move the stage 46 in the space S21. Reference symbol 136 denotes through holes for taking the tip 14 out from the tip supply device 15 placed in the space S221, into the space S21. Reference symbol 137 denotes a disposal opening for disposing the used tips 14.

In this case, in order to enable disposal of the tray members 51, a tray supply section 52 which supplies the unused tray members 51, and a tray collection section 53 which collects the used tray members 51 may be provided, so that the transfer robot 54 (tray supply device and tray collection device) can move them from the tray supply section 52 onto the stage 46, or from the stage 46 to the tray collection section 53.

The tray member 51 is for example a sterilized slab member, comprising concave portions (fixing portions) 51a for engaging with the culture containers 3, on the top surface, and an upright peripheral wall 51b all around the rim portion. Moreover, the bottom surface is provided with stepped portions 51c along two opposite sides, which form a gap between themselves and another adjacent tray member 51 underneath, or the floor, so that the tray members 51 can be lifted by the transfer robot 54 even in a state where they are stacked on the tray supply section 52. Furthermore, the bottom surface is provided with a fixing portion (not shown) which fixes the tray member 51 onto the mounting portion 46 of the shaker 21.

Similarly to the carrier robot 5, the transfer robot 54 is for example a multi-articulated robot comprising a fork shaped hand 55 on the tip end. The transfer robot 54 is designed to freely rotate the multi-joints to adjust the position of the hand 55 to the respective tray members 51 of the tray supply section 52, then insert the tip end of the fork shaped hand 55 into the stepped portions 51c provided on the tray member 51, and lift them, so as to carry the tray member 51.

By such a configuration, on the stage 46 where treatments such as medium exchange, stirring, and the like are performed on the culture container 3, then at the time of medium supply, stirring, or the like, the inside specimen, medium, or the like that flies out from the top opening of the culture container 3 to the outside, is received by the tray member 51 immediately underneath. Consequently, the range in which the media and specimen and the like are spattered can be kept within a range of the relatively narrow tray member 51, thereby preventing spattering over a wide range. Therefore, by merely exchanging the tray members 51, the space S2 in the culture treatment apparatus can be kept clean.

Moreover, since the tray member 51 is provided with the upright peripheral wall 51b all around the rim portion, even if a large amount of medium leaks from the culture container 3 onto the tray member 51, the peripheral wall 51b dams it up, preventing any further outward leakage.
Furthermore, since the tray member 51 is provided with the concave portions 51a for engaging the culture containers 3, then for example, when it is moved by the horizontal transfer mechanism 19, or when it is shaken by the operation of the shaker 21, or when the culture container 3 is tilted for exchanging medium, displacement of the culture container 3 with respect to the tray member 51 can be prevented.

In the above embodiment, each tray member 51 stacked on the tray supply section 52 is lifted by operation of the transfer robot 54 with the position of the hand 55 adjusted. However, instead of this, the tray supply section 52 side may be provided with a mechanism which pushes it up by a spring, so as to always supply the tray member 51 to the hand 55 at a fixed height position.

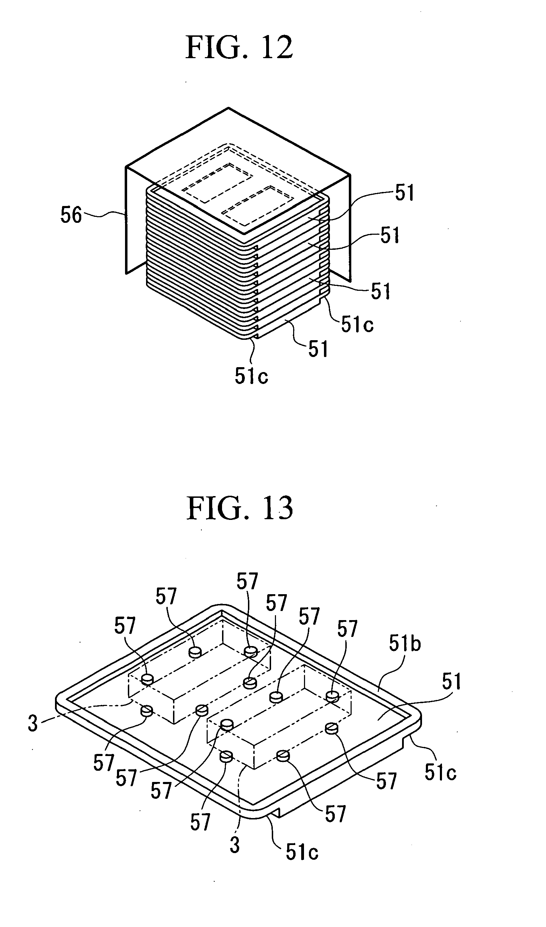

Moreover, as shown in FIG. 12, a cover 56 may be arranged to cover over the tray members 51 stacked on the tray supply section 52. As a result, dust floating toward the standby tray members 51 in the tray supply section 52 can be prevented from dropping and adhering thereonto.
Furthermore, the tray member 51 is provided with the concave portions 51a for engaging the culture containers 3. However, instead of these, as shown in FIG. 13, a plurality of pins 57 (fixing portions) that fit close against the side face of the culture container 3 may be provided.

The single hand 55 of the single transfer robot 54 is used to supply the unused tray members 51 and collect the used tray members 51. However, instead of this, hands may be separately used for supplying and for collecting. Also a transfer robot for supplying and a transfer robot for collecting may be separately provided. As a result, it becomes possible to more reliably prevent mixing with other types of specimens.

## Claims

1. A culture treatment apparatus comprising:
a treatment chamber in which is stored a treatment device which opens a lid of a culture container storing a specimen to perform a predetermined treatment on the specimen inside of the culture container; and
a sheet member arranged below the culture container, which covers a top surface of said treatment device.

2. A culture treatment apparatus according to claim 1 comprising:
a supply roll made from said sheet member in belt shape rolled into a roll shape;
a sheet supply device which sends out the sheet member from said supply roll; and
a sheet collection device which collects the used sheet member.

3. A culture treatment apparatus according to claim 2, wherein said sheet collection device comprises a roll-up device which rolls up the sheet member into a roll shape.

4. A culture treatment apparatus according to claim 2, wherein said sheet member is made from a liquid absorbable material.

5. A culture treatment apparatus according to claim 1 comprising an antiseptic solution supply device which impregnates an antiseptic solution into said sheet member.

6. A culture treatment apparatus according to claim 1, wherein said sheet member is made of a material having an antibacterial action.

7. A culture treatment apparatus according to claim 1 comprising a tray supply device which supplies a tray member onto a stage where a culture container is mounted, when a predetermined treatment is applied to a specimen in the culture container by opening a lid of the culture container storing the specimen.

8. A culture treatment apparatus according to claim 7 comprising a tray collection device which collects a used tray member after a treatment on a specimen in a culture container is completed, and the culture container is removed from the tray member.

9. A culture treatment apparatus according to claim 7, wherein said tray member is provided with a fixing portion which fixes a culture container on said tray member.

10. A culture treatment apparatus according to claim 7, wherein said tray member is provided with a peripheral wall which surrounds a culture container when it is mounted thereon.

11. An automatic culture apparatus comprising:
a culture chamber which stores a culture container storing a specimen, in a manner where it can be put in/taken out;
a treatment chamber which is arranged outside of said culture chamber, and which stores a treatment device which opens a lid of the culture container to perform a predetermined treatment on the specimen inside of the culture container; and
a carrier mechanism which carries the culture container between the treatment chamber and the culture chamber; wherein
at least either one of said treatment chamber and said culture chamber comprises a sheet member arranged below the culture container, which covers a top surface of said treatment device.
